# EUROPEAN PATENT SPECIFICATION

(11) **EP 1 059 880 B1**
(45) Date of publication and mention of the grant of the patent: **11.07.2007**
(21) Application number: 99908036.9
(22) Date of filing: 01.03.1999
(51) Int. Cl.: A61B 10/00

(54) **IMPACT-DAMPED BIOPSY INSTRUMENT**
STOSSGEDÄMPFTES BIOPSIE GERÄT
INSTRUMENT DE BIOPSIE LIMITEUR D'IMPACT

(30) Priority: 06.03.1998 SE 9800738
(43) Date of publication of application: 20.12.2000
(73) Proprietor: Bifos AB, 17007 Solna (SE)
(72) Inventor: WEILANDT, Anders, S-191 40 Sollentuna (SE); LINDGREN, Mikael, S-194 76 Upplands Väsby (SE)
(74) Representative: Rohmann, Michael
(86) International application number: PCT/SE1999/000286
(87) International publication number: WO 1999/044505

(56) References cited:
- EP-A1- 0 536 888
- EP-A1- 0 682 915
- US-A- 4 776 346
- US-A- 5 243 994
- US-A- 5 655 542

## Description

### AREA OF THE INVENTION

The present invention relates to a biopsy instrument and an apparatus for collecting tissue samples or the like from humans or animals, in particular by excising a segment from a tissue, including the biopsy instrument. The invention also relates to a loading assembly for a biopsy instrument and an impact damping element for biopsy instruments.

### BACKGROUND OF THE INVENTION

The invention departs from an apparatus for collecting tissue samples by excising a segment from tissue, and a corresponding method for harvesting of tissue samples described in U. S. Patent No. 5,655,542 (Weilandt). In this specification, the terms"proximal"and"distal"refer to the person extracting a biopsy sample. Thus, the proximal end of a biopsy apparatus is its rear end, pointing away from the patient.

The biopsy instrument of U.S. Patent No. 5,655,542 comprises a first part having an end with means for penetrating a tissue at the end, and a wall having an opening extending through the wall of the first part; and a second part adapted for being slidingly disposed on the first part, excision means for separating a segment of the tissue then penetrated by the first part provided on the second part such that sliding movement of the second part on the first part in one direction causes the excision means to pass through the opening in the wall of the first part thereby separating a segment of the tissue for extraction.

The method of harvesting biopsy samples of U.S. Patent No. 5,655,542 comprises the steps of
- injecting the biopsy instrument into tissue to a position proximal of a sample to be taken;
- displacing simultaneously the first part and the second part of the biopsy instrument for reception of the sample in the first part;
- displacing the second part to sever the sample from the tissue.

The first part may take the form of a canula and the second part that of a tube in which the canula is slidingly arranged. The tube is provided with a finger at its distal end, the finger entering an opening in the canula wall in the tissue-severing step when the tube is displaced in a distal direction in respect of the canula.

The joint displacement of the first part and the second part following the injection step is accomplished by spring means, in particular a steel coil. Compression steel coils have been found to work well in practice, except for very powerful springs providing high acceleration to the combination of the first and the second part. High acceleration is attractive since, thereby, the use of separate driving force for the second step becomes superfluous, the displacement of the second part in respect of the first part being accomplished through the inertia of the second part. Moreover high acceleration should provide for less variation in sample quality when using the instrument with a variety of tissues. The loading of a biopsy instrument provided with powerful spring means which have to be compressed in the loading procedure constitutes another problem.

EP-0536888-A1 discloses an automatic tissue sampling apparatus. The apparatus includes a needle having an inner needle and an outer needle. Two springs are provided to actuate the needle. Further, bumpers fabricated from a plastic or rubber material are provided to reduce noise and shock.

### OBJECTS OF THE INVENTION

It is an object of the invention to provide a biopsy instrument that can be reliably driven by strong spring means.

It is another object of the invention to provide a biopsy apparatus for use with strong spring means.

It is a further object of the invention to provide a means for damping the impact of a stylet or canula in a biopsy instrument.

It is an additional object of the invention to provide a loading assembly for loading a biopsy instrument, in particular one with a powerful compression spring.

### SUMMARY OF THE INVENTION

These and other objects of the invention are achieved by a biopsy instrument of the aforementioned kind as specified in claim 1, including means for damping oscillations of the first part in respect to the second part arising from the sudden stop of the first part at the end of the first step when hitting a stopper and that of the second part at the end of the second step. On impact the thin walled tubiform first and second parts become elongated in their axial direction. In general the canula and the finger tube of electropolished steel have a wall thickness of from 0.05 to 0.1 mm only while their length varies from about 15 to about 20 cm.

In experiments with strong steel coils oscillations of amplitudes of up to 1.15 mm were observed. In addition, the first and second part do not seem to oscillate in phase; they may even oscillate in opposite phase. The oscillations often damaged the finger, resulting in incomplete severing of the sample or breakage of the finger. The damage occurs by the finger hitting the distal rim of the opening in the tube wall. Thereby the finger is excessively bent or broken. According to the invention the oscillation damping means are provided by appropriate design of the stopper stopping the movement of the first part. In the preferred embodiment of the '542 patent the first stopping element is the plunger stop 960 modified according to the teaching of the present invention. By using a powerful steel compression spring the actuator coil 94 can be dispensed with. The displacement of the second part in relation to the first part then is caused entirely by inertia.

It is preferred for the steel coil of the biopsy instrument to exert a force of 35 N and more in a compressed state. Particularly preferred is a coil exerting a force of at least 50 N, most preferred of at least 65 N.

A reduction of oscillation by damping so as to keep oscillation of the first part in respect of the second part below 0.4 mm provides a satisfactory result and is preferred. Even more preferred is to keep oscillation within 0.35 mm, even within 0.30 mm and less.

A biopsy instrument according to the invention combines a powerful steel compression spring of the kind described above, an impact damping means to reduce oscillation of a canula in respect of a finger tube slidingly disposed on the canula arising on impact of the canula holder on a stopper, and a design dispensing with the use of spring means to displace the finger tube in a distal direction after the canula has been stopped by the stopper.

The impact damping means of the invention can also be advantageously applied to stop (decelerate) canulas and stylets in biopsy instruments other than that of US 5,655,542, in particular canulas and stylets accelerated by a powerful compression spring, such as one which can be compressed to more than 35 N, preferably more than 50 N, most preferred more than 65 N. The damping means of the invention comprises an ABS polymer having a yield tensile strength above 35 Mpa and an lzod impact strength of more than 325 J/m or any other medical grade polymer meeting these requirements.

The loading assembly of a biopsy apparatus incorporating, in a housing, the biopsy instrument according to the invention comprises a loading arm swivellingly attached at its one end to the housing, a intermediate arm swivellingly attached at its one end to the loading arm and at its other end to a catch holding the canula holder during tensioning and in a tensioned position in respect of a tension spring or during compression or in a compressed position in respect of a compression spring (the latter being preferred), the catch being displaceable in a proximal direction against the resistance of the spring and being adapted to be held there for intentional release. It is obvious that this loading assembly can also be advantageously used for tensioning or compressing spring means arranged for displacement of one or several elements of a biopsy apparatus different from that of the invention.

Further advantages of the present invention will become obvious from the study of a preferred but not limiting biopsy instrument and a corresponding apparatus according to the invention which is illustrated in a drawing of which the individual fugures show:
- Fig. 1: a canula with its holder, in a perspective view from a distal viewpoint, only the rear (proximal) portion of the canula being shown;
- Fig. 2: a finger tube with its holder, in the same view as in Fig 1, only the rear (proximal) portion of the finger tube being shown;
- Fig. 3: the canula and finger tube holders, in a mounted state before and during the first step, in the same view as in Figs. 1 and 2;
- Fig. 4: the canula, the finger tube, and their respective holders, in a mounted state in the same view as before, at the end of the second step;
- Fig. 5: a stopper, in the same view as before;
- Fig. 6: the canula holder, the finger tube holder, and the stopper, in a mounted state at the end of the second step, in the same view as before;
- Fig. 7: the assembly of Fig. 6 before and during the first step, in a symmetric longitudinal section;
- Fig. 8: a biopsy apparatus incorporating the biopsy instrument of Figs. 1-7, in a loaded condition and in a symmetric longitudinal section;
- Fig. 9: the apparatus of Fig. 8, in a released condition at the end of the first step, in the same view as in Fig. 8;
- Fig. 10: the apparatus of Fig. 8, in a released condition at the end of the second step, in the same view as in Fig. 8; Fig. 11 the apparatus of Fig. 8, after withdrawal of the finger from the canula but before sample expulsion and loading, in the same view as in Fig. 8; Fig. 12a the catch of the loading assembly of the apparatus of Fig. 8, in a plan view from above; Fig. 12b the catch of Fig. 12a, in the same view as the apparatus of Figs. 8-11.

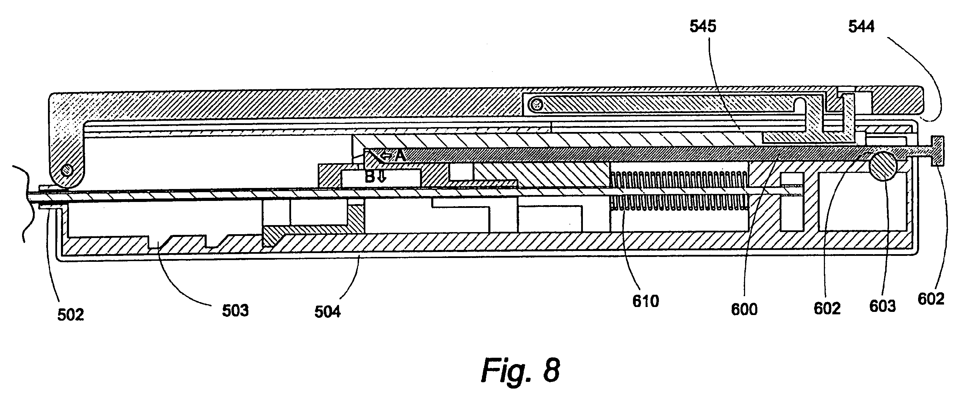

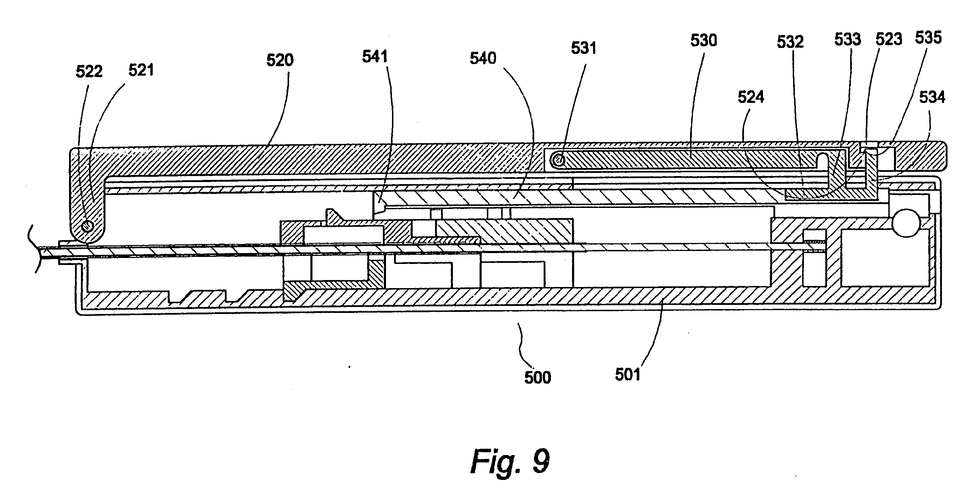

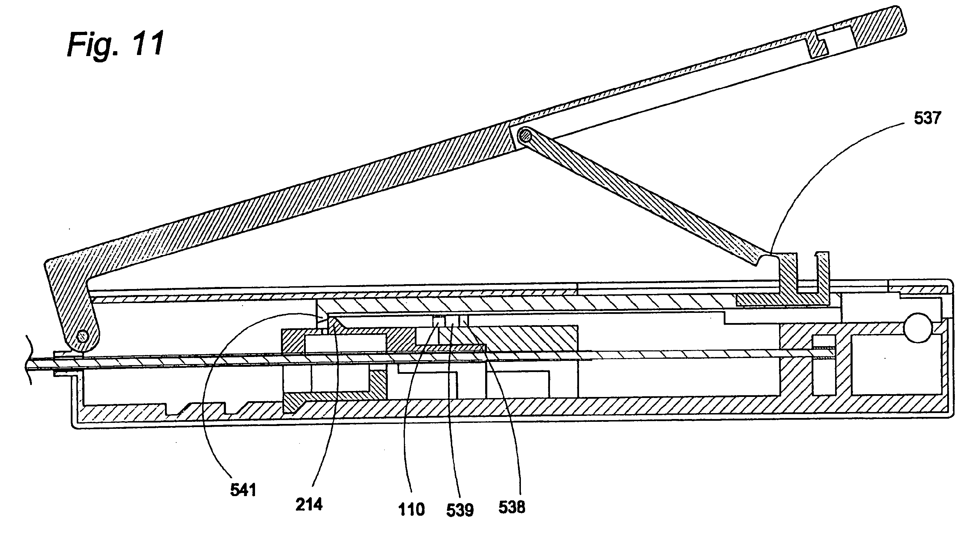

Indications of lateral direction such as 'from above', 'underside', 'top side' are defined by the view of Figs. 8-11. The steel coil compression spring is only shown in Fig. 8.

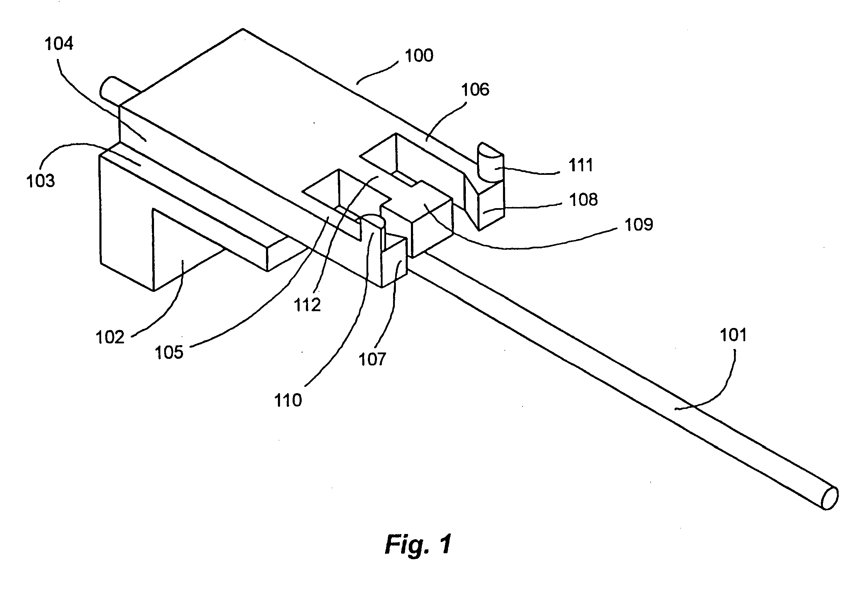

The biopsy instrument illustrated in Figs. 1-7 comprises a canula 101, a finger tube 201, and a stylet 301 of the same kind as in U. S. Patent No. 5,655,542 where the design and function of these elements is explained in detail and thus need not to be repeated here. In some of the figures of the present specification rear (proximal) end portions of the canula 101, the finger tube 201, and the stylet 301 are shown to illustrate their relationship with their respective holders 100, 200,300, each made in one piece of a polymer material, preferably an ABS polymer (acrylonitrile-butadiene-styrene copolymer) or another polymer of high impact and tensile strength.

Fig. 1 shows the rear portion of the canula 101 mounted to its holder 100 by gluing. The flat and about rectangular top section of holder 100 has a sturdy and somewhat wider foot section integral with its underside which has a transverse flat impact face 102 facing in a distal direction. By having a width in excess of that of the rectangular top section a pair of upwardly facing slide ways of which only one, 103, is shown are formed on the foot section. The rectangular top section is provided with laterally facing slide ways of which only one, 104 is shown. Slide ways 103 and 104 and their not illustrated counterparts form a pair of mirroring L-shaped guide ways cooperating with a pair of guide rails protruding from opposite sides of the inner wall of a housing (not shown). The front (distal) portion of the top section ends in a pair of slightly flexible arms 105, 106 encompassing a rectangular wide through opening into which the neck 112 of a T-formed displacement limiter protrudes from the distally facing rim of the opening. At their free ends the arms 105 and 106 are widening inwardly to form teeth 107, 108 carrying on their upper faces inwardly rounded stop pins 110, 111 the function of which will be explained below.

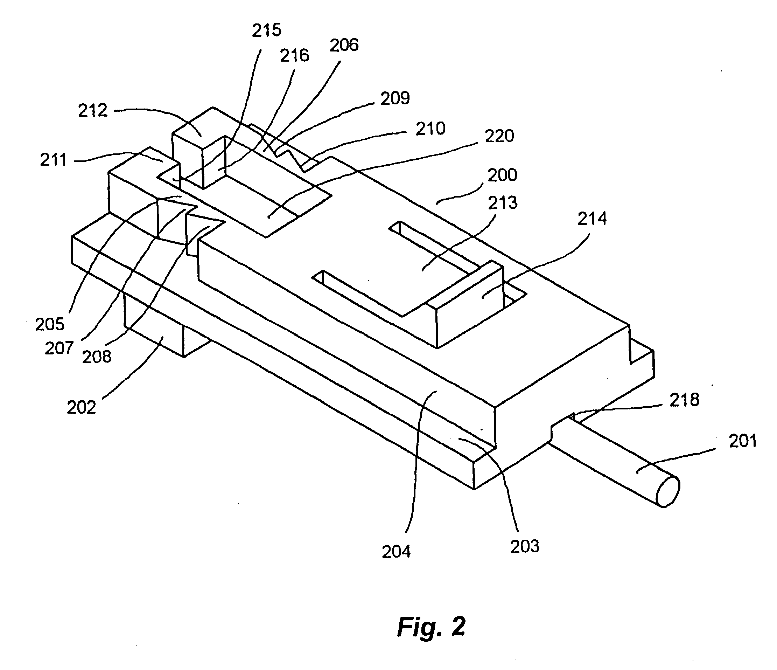

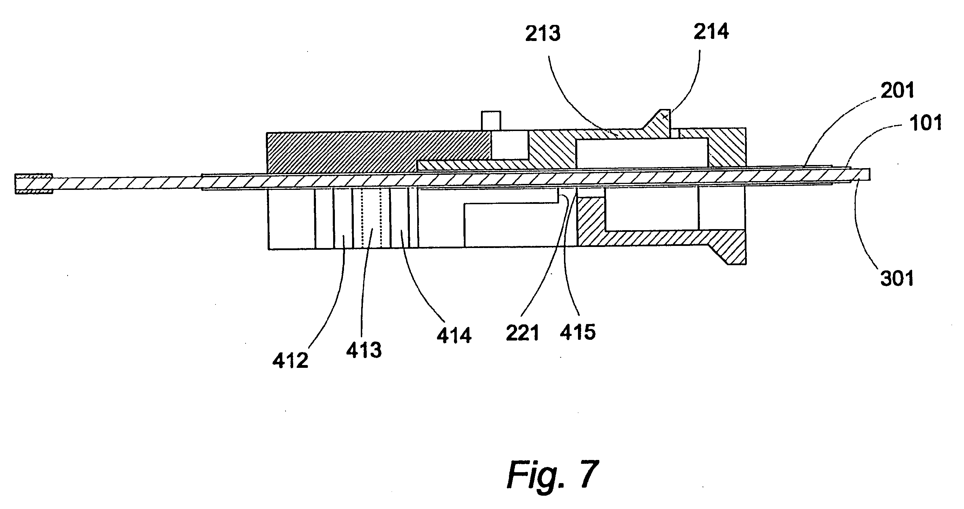

Fig. 2 shows the rear portion of the finger tube 201 disposed in a channel 218 of a holder 200 to which it is mounted by gluing. The rear (proximal) end of the finger tube does not protrude from the rear end of the holder 200. The foot section comprises a heel 202 and a rectangular base of same width as the foot section of the canula holder 100. On top of the rectangular base is disposed an about rectangular top section. At its rear end the top section comprises two arms 205, 206 integral with the base, encompassing a rectangular opening the depth of which is delimited by the top face 209 of the rectangular holder base. The outer (lateral) faces of arms 205, 206 are provided with pairs of indentations 207, 208; 209, 210 for cooperation with teeth 107, 108 of canula holder 100. At their rear free ends arms 205, 206 have inwardly extending hook portions 211, 212 leaving open a passage wide enough to fit around the neck 112 of the displacement limiter. The holder 200 is provided with two pairs of slide ways forming L-shaped guide ways similar to those of holder 100. Of them only one pair, 203; 204 is shown. The left and the right guide ways of holders 100 and 200 are disposed in line. As is evident from Fig. 7 the top section of the holder 200 has a hollow portion distally off arms 205, 206, the top wall of which is cut in form of a tongue 213. The tip 214 of tongue 213 projects above the upper surface of the top wall. The tongue 213 is somewhat flexible and can be depressed into the hollow space beneath it.

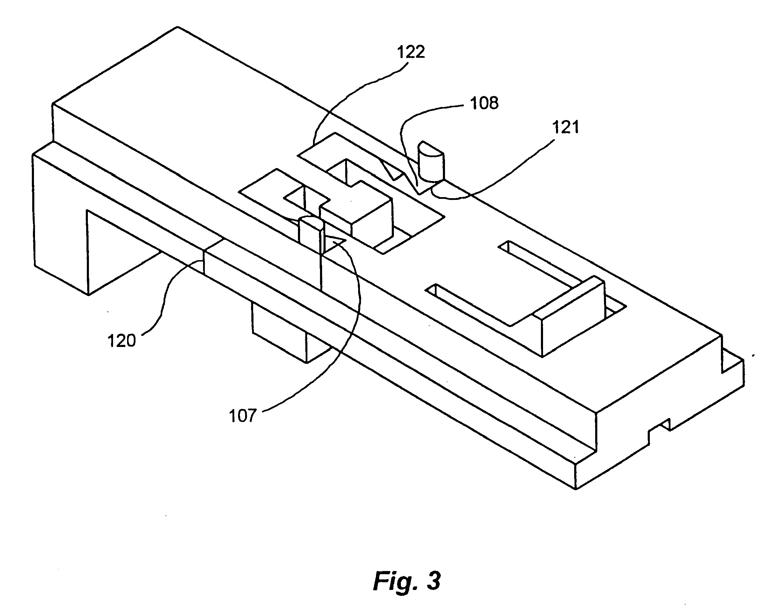

Fig. 3 shows the canula 101 with its holder 100 and the finger tube 201 with its holder 200 in an assembled state before and during the first step in which both travel together. Whereas the canula holder 100 is pushed forward (in a distal direction) by a strong steel coil (not shown; compressed to 70 N) affecting its rear (proximal) end, the finger tube holder 200 is pushed forward by the canula holder 100 with which it is in abutment at 120, 121, 122. Canula holder teeth 107, 108 engage with the respective distal indentation 208, 210 of the finger tube holder 200 to avoid unintended forward movement of the finger tube 201.

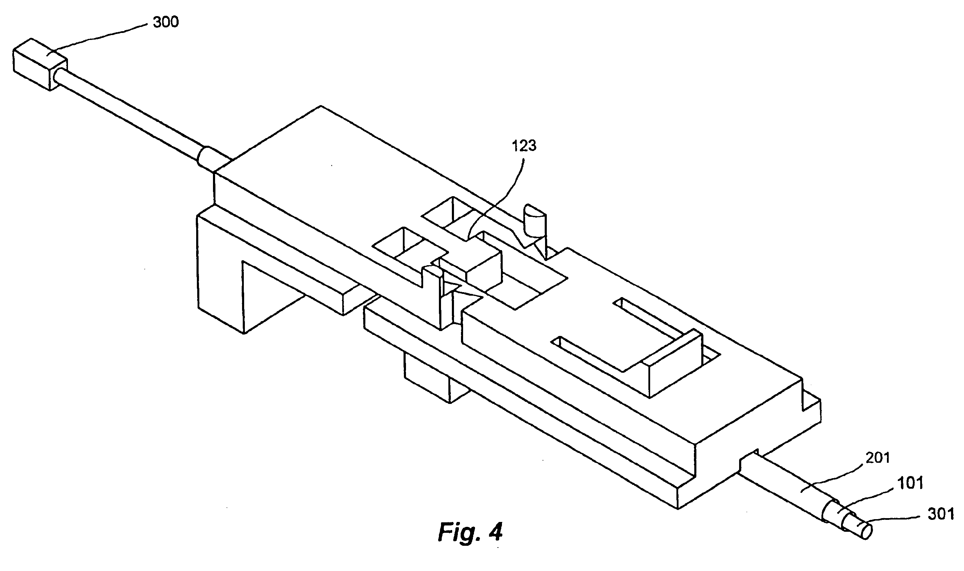

Fig. 4 shows the same arrangement after the forward travel of canula holder 100 has come to an end while the finger tube holder continues its movement by inertia, only to be stopped by the rear side of the transverse bar of the T-formed displacement limiter 112 abutting faces 215, 216 of hooks 211, 212, respectively. For reasons of balance an additional stop is provided by a front face 221 of the distal heel portion cooperating with a rearward facing wall 415 of the stopper 400 (see below; see Fig. 7 which however illustrates the assembly before or during the joint displacement of the canula 101 and the finger tube 201). In this figure the canula 101, the finger tube 201, and the stylet 301 are shown except for their distal ends. Stylet holder 300 is fixed to the housing (not shown) and thus does not move.

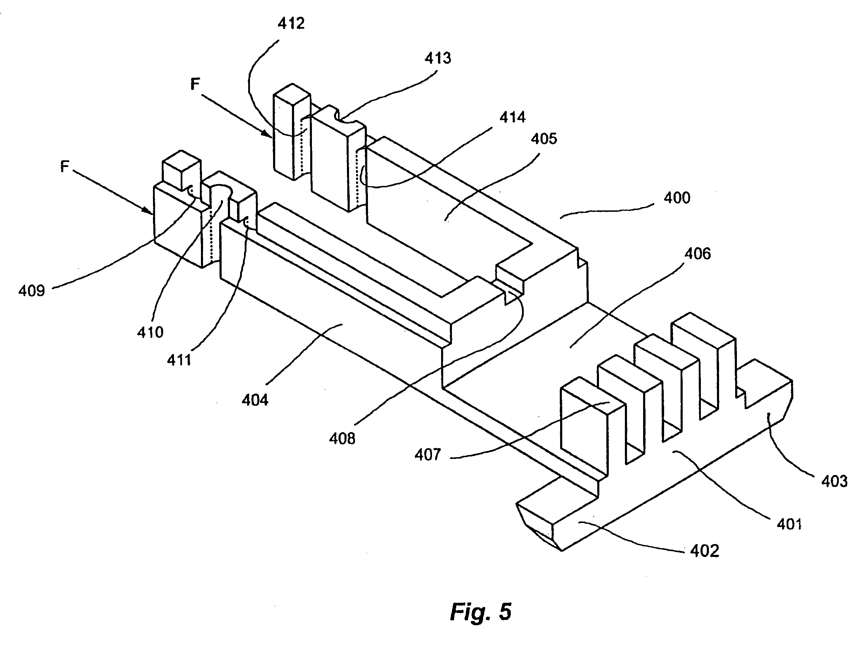

Fig. 5 shows a fork-like stopper 400 for restricting the travel of the canula 101 and the finger tube 201. By arms 402, 403 of the front-end fork shaft handle 401 protruding at opposite sides from the housing (not shown) the stopper 400 can be set at several positions along the canula/stylet axis to select one of a number of predetermined tissue penetration depths. The prongs 404, 405 of the fork are directed rearwards to meet the impact (indicated by 'F' in the drawing) of the canula holder (by foot face 102) at their proximal ends 419, 420. The impact is damped by hemi-cylindrical recesses 409,410,411; 412,413,414 alternating at the inner and outer lateral faces of each prong. On impact the prongs 404, 405 are compressed in an axial direction and deformed by a sort of bending due to these recesses. ABS polymers are particularly well suited to the purpose of shock absorbers since they have a high softening point and excellent impact strength as well as a good yield tensile strength (toughness). One may say that they combine, by their chemical nature, the good yield tensile strength of general purpose (low impact) polystyrene with the high impact strength of rubbery polymers, such as butadiene rubber. The holders 100, 200, 300 and the stopper 400 are produced by injection molding. In combination with a sort of serpentine geometry of the prong end portions this provides for good damping of the oscillatory movement of the canula 101 and the finger tube 201 with their holders 100, 200. With the teaching of the invention in mind the person skilled in the art will be able to produce the damping effect also by modifications of the stopper design other than hemi-cylindrical recesses, for instance by a zigzag design, by bores, etc.

Proper damping (in respect to spring force, etc.) can be determined by experiments in which the depth and/or the number recesses is varied. In the preferred embodiment according to the invention described above a steel coil compressed to 70 N was used. The oscillation amplitude of the canula 101 in respect of the finger tube 201 was found experimentally to be in the range from 0.30 to 0.35 mm. In a device identical with that of the invention except for the hemi-cylindrical recesses 409, 410 ,411 ; 412, 413, 414 which had been omitted a relative oscillation amplitude of from1.0 to 1.1 mm was determined experimentally. In this case excessive finger bending or breakage was observed. By using steel coils compressed to a force of 30 N or less oscillation could be kept within acceptable limits.

Between the handle 401, which is provided with stiffening ribs 407, and the prongs 404, 405 the shaft has a flat transitional portion 406 which can be bent to allow the fork shaft handle 401 of the stopper 400 disposed in a latch arranged in the housing wall to be moved in distal/proximal direction between three positions, for instance to latch 503 which position provides for maximal stroke length. The ends 402, 403 of the fork shaft handle 401 protrude through slits at opposite sides of the housing 500 and are thus maneuverable from outside. A central groove 408 cooperates with a corresponding groove 218 in the finger tube holder 200 and another one (not shown) in the canula holder 100 is provided for receiving the stylet/canula/finger tip tube assembly.

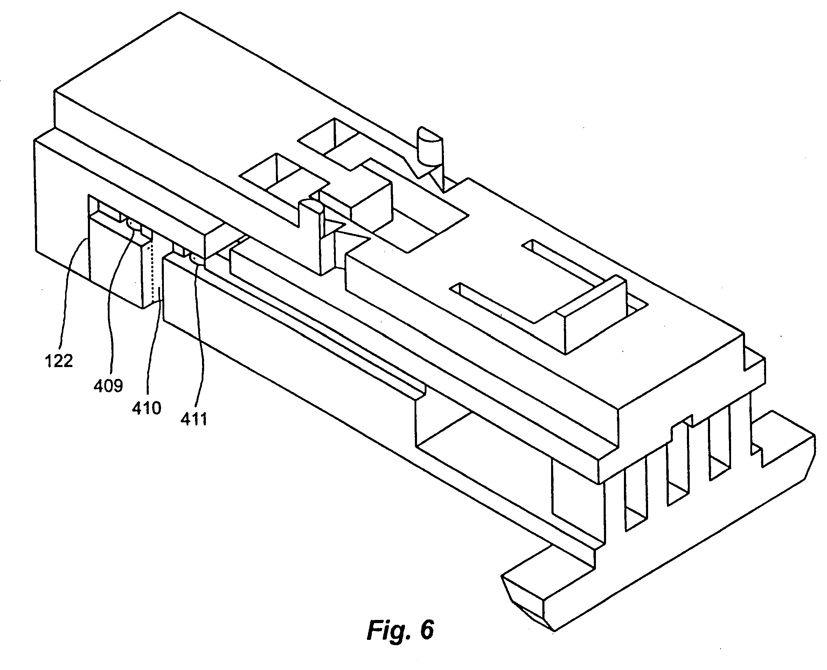

In Fig. 6 the stopper 400 is shown mounted with the stylet/canula/finger tube assembly of Fig. 4 at the end of the movement of the finger tube 201. Fig. 7 is a corresponding longitudinal section.

The biopsy apparatus 500 of Figs. 8-12b comprises the biopsy instrument of Figs. 1-7 in a housing 500 consisting of two substantially symmetrical halves sectioned perpendicular to the larger faces of the holders 100, 200 and of the catch 540. Only one half is shown. The housing which has about the form of a rectangular bar can be of a suitable polymer, for instance polycarbonate, of metal, such as aluminum. The housing halves fit by appropriate design of rims 501 and other elements, such as snaps (not shown). The halves can be connected by gluing, welded, or similar.

The holder 300 of stylet 301 is firmly fixed at the housing and can only move with it. The stylet 301, the canula 101 and the finger tube 201 which are disposed about centrally in the housing 500 in a distal direction protrude from the front wall after passage through an opening (at 502) of a diameter slightly greater than the outer diameter of the finger tube 201. The walls of the housing 500 halves have inner profiles functioning as guide rails for holders 100 and 200 which can move forth and back in the housing to the extent permitted by other elements, such as the stopper 400 and the spring 610.

The housing is provided with a loading assembly by which holders 100, 200 are brought to a 'loaded' position from which they can be released for tissue sampling. To provide for easy loading by which the sturdy spring coil 601 has to be compressed the assembly makes use of three elements, a loading arm 520, a intermediate arm 530, and a catch 540 holding the canula holder 100 during compression or in a compressed (loaded) position. With its hook-like front end 541 the catch 540 grips the tip 214 of the finger tube holder's 200 tongue 213, as shown in Fig. 8 where the apparatus is in a loaded state. The canula holder 100 in turn is held in position by some of its front faces abutting rear faces of finger tube holder 200, such as at 120 (Fig. 3). The canula holder/finger tube holder assembly 100, 200 can be released from the loaded state by an oblong trigger 600 disposed between the catch 540 and canula holder/finger tube holder assembly 100, 200. The rear end of trigger 600 forming a release button 602 passes through an opening in the rear wall of the housing 500. By pressing the release button 602 the trigger 600 is pushed forward (direction A in Fig. 8), therby dislocating downwards, by its slanting front end abutting the slanting rear face of tongue tip 214 (direction B in Fig. 8), thereby releasing the canula holder/finger tube holder assembly 100, 200. The biopsy apparatus can be secured in a loaded position by a transversely displaceable locker pin 603 disposed near the rear end of the housing in through bores 602 of the housing. When inserted the locker pin 603 cooperates with an arc-formed cutout of the trigger 600.

Prior to samling the cannula 101 is inserted into the tissue to a depth at which a sample is intended to be collected. Injection is made easy by the use of a canula of small diameter having a thin wall and a sharp cutting edge.

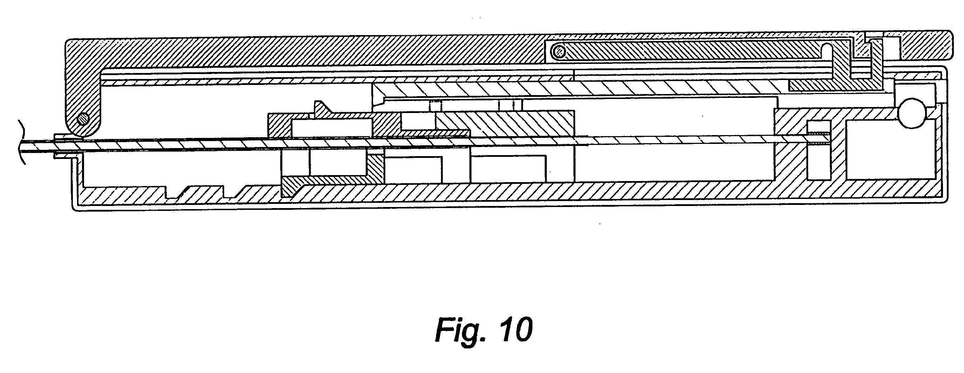

In the first sampling step the canula holder 100 and the finger tube holder 200 are dislocated together in a distal direction until the canula holder foot face 102 hits the rear end of the stopper 400 (at arrow heads F in Fig. 5). In the second sampling step the finger tube holder 200 with the finger tube 201 continues to travel by inertia until stopped by rear faces of the cannula holder (at 123; Fig. 4) and the stopper (at 415; Fig. 7). At the end of the second step (Fig. 10) the finger (not shown) of the finger tube 201 has entered the interior of the canula 101 through a wall opening, thereby cutting off an about cylindrical tissue sample disposed in the canula. On withdrawal of the biopsy apparatus the tissue sample stays in the canula and can be recovered.

By pushing the rear end 544 of the loading arm 520 away from the housing 500 the transverse bar 521 of the front end of the L-shaped loading arm 520 is made to swivel around a pivot 522 by which it is fixed at the housing. This releases the rear end of the loading arm 520 from a snap connection comprising a snap hook 535 of the upper transverse bar of an F-formed intermediate arm end portion engaging with a shoulder 524 of the loading arm 520 pertaining to the wall of an opening 523 therein. The vertical bar 532 of the F-shaped intermediate arm 530 portion is slidingly disposed in the housing 500. The lower transverse bar 533 protrudes from the housing 500 trough a wide slit extending over a substantial portion of the housing 550 in a distal direction from close its rear end. Near its other end the intermediate arm 530 is fastened to the loading arm 520 rotatably about a link 531. A further joint 537 is provided in the intermediate arm 530 where its straight portion extending from the link 531 is joined to the lower transverse bar 533. This joint 537 is obtained by the provision of a recess which makes the relatively thin (1-2 mm) portion of reduced thickness easy to bend; a flexible polymer like polyethylene or nylon is an appropriate material for the intermediate arm 530.

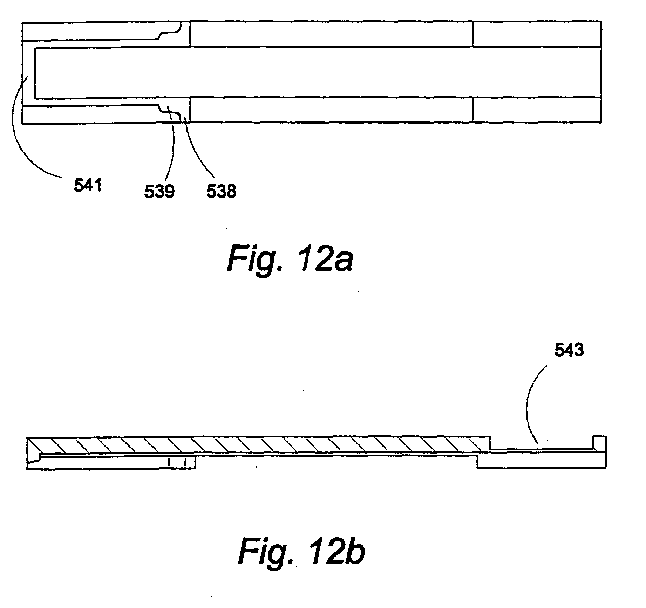

When loading arm 520 is pushed or pulled from the housing 500 the F-shaped intermediate arm portion travels in a distal direction with the catch 540 of which it engages a recess 543 (Fig. 12b). The front end hook portion 541 of the catch eventually overrides the tongue tip 214 by forcing it to bend downwards through pressure applied against its the slanting rear face. After engagement of the hook portion 541 with the tongue tip 214 further movement of the catch 540 in a distal direction is prevented by the stops 538 on the catch 540 abutting the inwardly rounded stop pins 110, 111 of the canula holder 100. Just before reaching the stops 538 the stop pins 110, 111, and with them the resiliently flexible arms 105, 106 of the canula holder 100, are pressed outwards by shoulders 539 of lateral, outward facing guide walls (Fig. 12a) and remain in that position when abutting stops 538. The catch 540 now has reached its extreme distal position. Further movement of the finger tube holder 200 in respect of the canula holder 100 is prevented by their abutment at 123 (Fig. 4). Since the shoulders 539 slant sligthly inwardly in a direction away from the stops 538 the stop pins 110, 111 with the arms 105, 106 are pressed rearwards. Thereby the teeth 107, 108 of the flexible arms 105, 106 disposed in the proximal indentations 208, 210 of finger tube holder arms 205, 206 become disengaged whereby the finger tube holder 200 is unlocked and carried forward until it is stopped by the canula holder 100 while the teeth 107, 108 snap into the distal indentations 207, 209. In Figs. 8-11 the lines indicating the position of stop pin 110, the shoulder 539, and the stop 538 are dotted.

By carefully moving, in the following step, the loading arm 540 towards the housing 500 the canula 101 and the finger tube 201 are retracted while the sample is expelled by the stylet 301. The biopsy apparatus is now ready for reloading to prepare it for removal of another sample of tissue. Arm 520 is pressed towards the housing 500 until the hook 535 of the intermediate arm enters the opening 523 of the loading arm 520 and engages the shoulder 532 to form a snap connection. At the same time the free end portion 532 of the intermediate arm 530 moves rearwards and compresses the coil spring 610 via intermediate holders 100, 200 of the biopsy instrument. The flat trigger 600 is only active in triggering release and, therefore, has been omitted from the drawings except for Fig. 8.

The construction of the loading assembly is also advantageous in that the loading arm 520 with the apparatus in a loaded position forms a single body with the housing 500 which is comfortable to hold and maneuver, and which is safe. It is also advantageous through its reduction of the force needed for compressing the spring coil. It is obvious to the person skilled in the art that the loading assembly also could be used with a strong tension spring which, for other reasons, is however not as attractive in the present context as a compression spring.

The person skilled in the art will have no difficulty to modify the invention by various combinations of its features without departing from its teaching.

## Claims

1. A biopsy instrument for extracting a segment from tissue, the biopsy instrument comprising:
a first part (101) having an end with means for penetrating a tissue at the end, and a wall having an opening extending through the wall of the first part (101);
a second part (201) adapted for being slidably disposed on the first part (101), excision means for separating a segment of the tissue then penetrated by the first part (101) provided on the second part (201) such that sliding movement of the second part (201) on the first part (101) in one direction cause the excision means to pass through the opening in the wall of the first part (101) thereby separating a segment of the tissue for extraction;
steel spring means (610) exerting, when compressed, a force of at least 35 N, said steel spring means (610) being arranged for displacing the first and second part (101, 201) in a distal direction; and
**characterized by** impact damping means (400) of ABS or of a polymer having physical properties similar to ABS, the impact means (400) including two prongs (404, 405), each prong having at least two recesses (409-414), one on each side, to reduce the oscillation of the first part (101) in relation to the second part (201) at stoppage of the first part (101).

2. The biopsy instrument of claim 1 provided with impact damping means (400) designed to keep oscillation of the first part (101) in respect to the second part (201) within 0.4 mm.

3. The biopsy instrument of claim 1, wherein the steel spring means (610) exerts a force of at least 50 N.

4. The biopsy instrument of claim 1, wherein the steel spring means (610) exerts a force of at least 65 N.

5. The biopsy instrument of claim 1, wherein the damping means (400) are capable of keeping the oscillations within 0.3 mm.

6. The biopsy instrument of claim 1, wherein the sliding movement of the second part (201) is caused by inertia.

7. The biopsy instrument of claim 1, wherein the damping means (400) are integral with means for stopping the movement of the first part (101).

## Patentansprüche

1. Biopsieinstrument zum Extrahieren eines Segments aus Gewebe, wobei das Biopsieinstrument Folgendes umfasst:
einen ersten Teil (101), der ein Ende mit Mitteln, um ein Gewebe am Ende zu durchdringen, aufweist, und eine Wand, die eine Öffnung aufweist, die sich durch die Wand des ersten Teils (101) erstreckt;
einen zweiten Teil (201), der angepasst ist, um schiebbar auf dem ersten Teil (101) angebracht zu sein, Entfernungsmittel zum Abtrennen eines Segments des Gewebes, welches dann vom ersten Teil (101) durchdrungen wird, die auf dem zweiten Teil (201) bereitgestellt sind, derart dass eine Schiebebewegung des zweiten Teils (201) auf dem ersten Teil (101) in einer Richtung dazu führt, dass die Entfernungsmittel durch die Öffnung in der Wand des ersten Teils (101) hindurch passieren, wobei sie ein Segment des Gewebes zur Extraktion abtrennen;
Stahlfedermittel (610), welche, wenn sie komprimiert sind, eine Kraft von mindestens 35 N ausüben, wobei die Stahlfedermittel (610) so angeordnet sind, dass sie den ersten und zweiten Teil (101, 201) in eine distale Richtung verschieben; und
charakterisiert durch Anschlagdämpfungsmittel (400) aus ABS oder einem Polymer, welches ABS-ähnliche physikalische Eigenschaften aufweist, wobei das Anschlagmittel (400) zwei Zinken (404, 405) beinhaltet, wobei jede Zinke mindestens zwei Aussparungen (409-414) aufweist, jeweils einen auf jeder Seite, um die Schwingung des ersten Teils (101) in Bezug auf das zweite Teil (201) beim Anhalten des ersten Teils (101) zu reduzieren.

2. Biopsieinstrument nach Anspruch 1, welches mit Anschlagdämpfungsmitteln (400) bereitgestellt ist, die entworfen sind, um die Schwingung des ersten Teils (101) bezüglich des zweiten Teils (201) innerhalb 0,4 mm zu halten.

3. Biopsieinstrument nach Anspruch 1, wobei das Stahlfedermittel (610) eine Kraft von mindestens 50 N ausübt.

4. Biopsieinstrument nach Anspruch 1, wobei das Stahlfedermittel (610) eine Kraft von mindestens 65 N ausübt.

5. Biopsieinstrument nach Anspruch 1, wobei die Dämpfungsmittel (400) die Schwingung innerhalb 0,3 mm halten können.

6. Biopsieinstrument nach Anspruch 1, wobei die Schiebebewegung des zweiten Teils (201) durch Trägheit verursacht wird.

7. Biopsieinstrument nach Anspruch 1, wobei die Dämpfungsmittel (400) mit Mitteln zum Anhalten der Bewegung des ersten Teils (101) ein Teil bilden.

## Revendications

1. Instrument de biopsie destiné à extraire un segment de tissu, l'instrument de biopsie comprenant:
une première partie (101) présentant une extrémité avec des moyens pour pénétrer dans un tissu sur l'extrémité et une paroi présentant une ouverture s'étendant à travers la paroi de la première partie (101);
une seconde partie (201) apte à être disposée de façon coulissante sur la première partie (101), des moyens d'excision pour séparer un segment du tissu alors pénétré par la première partie (101) disposée sur la deuxième partie (201), de sorte que le mouvement coulissant de la deuxième partie (201) sur la première partie (101) dans une direction fait en sorte que les moyens d'excision traversent l'ouverture dans la paroi de la première partie (101), séparant ainsi un segment du tissu pour l'extraction ;
des moyens de ressort en acier (610) exerçant, lorsqu'ils sont comprimés, une force d'au moins 35 N, lesdits moyens de ressort en acier (610) étant aptes à déplacer la première et seconde partie (101, 201) dans une direction distale ; et
**caractérisé par** des moyens amortisseurs d'impact (400) d'ABS ou d'un polymère ayant des propriétés physiques similaires à l'ABS, les moyens d'impact (400) comprenant deux pinces (404, 405), chaque pince ayant au moins deux évidements (409, 414) de chaque côté pour réduire l'oscillation de la première partie (101) par rapport à la seconde partie (201) lors de l'immobilisation du mouvement de la première partie (101).

2. Instrument de biopsie selon la revendication 1, muni de moyens d'amortissement d'impact (400), conçu pour maintenir l'oscillation de la première partie (101) par rapport à la seconde partie (201) à l'intérieur de 0,4 mm.

3. Instrument de biopsie selon la revendication 1, dans lequel les moyens de ressort en acier (610) exercent une force d'au moins 50 N.

4. Instrument de biopsie selon la revendication 1, dans lequel les moyens de ressort en acier (610) exercent une force d'au moins 65 N.

5. Instrument de biopsie selon la revendication 1, dans lequel les moyens d'amortissement (400) sont capables de maintenir les oscillations à l'intérieur de 0,3 mm.

6. Instrument de biopsie selon la revendication 1, dans lequel le mouvement coulissant de la seconde partie (201) est provoqué par l'inertie.

7. Instrument de biopsie selon la revendication 1, dans lequel les moyens d'amortissement (400) sont solidaires de moyens pour immobiliser le mouvement de la première partie (101).
